(19) Europäisches Patentamt
European Patent Office
Office européen des brevets

(11) **EP 3 312 584 B1**

(12) **EUROPEAN PATENT SPECIFICATION**

(45) Date of publication and mention
of the grant of the patent:
**08.01.2020 Bulletin 2020/02**

(51) Int Cl.:
***G01N 1/22*** *(2006.01)*

(21) Application number: **15895690.4**

(86) International application number:
**PCT/JP2015/083583**

(22) Date of filing: **30.11.2015**

(87) International publication number:
**WO 2016/203671 (22.12.2016 Gazette 2016/51)**

(54) **CONTINUOUS MEASUREMENT METHOD FOR HYDROGEN GAS CONCENTRATION AND HYDROGEN GAS CONCENTRATION MEASUREMENT DEVICE USED IN SAME**

KONTINUIERLICHES MESSVERFAHREN FÜR WASSERSTOFFGASKONZENTRATION UND DARIN VERWENDETE VORRICHTUNG ZUR WASSERSTOFFGASKONZENTRATIONSMESSUNG

PROCÉDÉ DE MESURE CONTINUE DE CONCENTRATION EN GAZ HYDROGÈNE ET DISPOSITIF DE MESURE DE CONCENTRATION EN GAZ HYDROGÈNE UTILISÉ DANS CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2015 JP 2015122854**

(43) Date of publication of application:
**25.04.2018 Bulletin 2018/17**

(73) Proprietor: **Pureron Japan Co., Ltd.**
**Iwaki-shi, Fukushima 970-1144 (JP)**

(72) Inventors:
• **NAKAJIMA, Hidetoshi**
**Iwaki-shi**
**Fukushima 970-1144 (JP)**

• **TAGUCHI, Koji**
**Iwaki-shi**
**Fukushima 973-8408 (JP)**

(74) Representative: **Schön, Christoph**
**Dr. Schön, Neymeyr & Partner mbB**
**Bavariaring 26**
**80336 München (DE)**

(56) References cited:
WO-A1-2015/059810    WO-A1-2015/059810
WO-A1-2015/074990    JP-A- H 085 618
JP-A- H0 495 752     JP-A- H0 666 690
JP-A- H02 140 649    JP-A- S54 121 196
JP-A- S57 179 726    JP-A- 2006 138 653
JP-A- 2008 180 524   JP-A- 2011 007 758
JP-U- 3 197 572      JP-U- S57 164 459

## Description

## Technical Field

The present invention relates to a continuous measurement method for hydrogen gas concentration and a hydrogen gas concentration measurement device used in the method.

## Background Art

As a conventional method for measuring the concentration of hydrogen gas (dissolved hydrogen gas) contained in water in which hydrogen gas dissolves (hereinafter abbreviated as hydrogen water), a method of measuring a hydrogen-ion concentration (pH) is known, for example. As a device used in the method, a device including a reference electrode and a work electrode is known. With the device, the reference electrode and the work electrode are immersed in a liquid to be measured, and the pH is determined on the basis of the outputs from the respective electrodes (for example, see Patent Literature 1).

## Citation List

## Patent Literature

Patent Literature 1: Japanese Patent Application Laid-Open No. 2012-163531
WO 2015/059810 A1 discloses a dissolved gas concentration measurement device and a dissolved gas concentration measurement method that make it possible to easily and reliably carry out continuous measurement of the concentration of a dissolved gas dissolved in a liquid. In the dissolved gas concentration measurement device, a first container has a constant free space, which is the space not occupied by a liquid, and an air intake hole through which external air can flow into the free space. Dissolved gas that is separated from the liquid mixes with the external air that flows in from the air intake hole and is supplied to a concentration sensor in the form of a gas mixture.
WO 2015/074990 A1 discloses an apparatus for detecting gas in a high-voltage device filled with an insulating medium which comprises an inlet for introducing a carrier gas and an outlet for discharging a carrier gas; at least one gas sensor for detecting a gas; a first pump for conveying the carrier gas in the apparatus; a membrane which at least consists of at least one semipermeable material, is at least partially surrounded by the insulating medium and is at least partially subjected to a flow of the carrier gas; a second pump for conveying the carrier gas into the apparatus and for conveying the carrier gas out of the apparatus; wherein there is no valve which can be used to convey the carrier gas into the apparatus or out of the apparatus.
Furthermore, JPH 085618 A discloses an apparatus for detecting a gas component in water, wherein a supply gas is pumped through a gas permeation membrane and the gas component is detected by a detecting part.

## Summary of Invention

## Technical Problem

The above method of measuring a hydrogen-ion concentration (pH) is, however, essentially a method of measuring the electric potential of hydrogen ions in a liquid to be measured, but is not a method of directly measuring the concentration of hydrogen gas as molecular hydrogen, and thus has a disadvantage of failing to reliably measure the concentration of hydrogen gas.
The present invention is therefore intended to overcome such a disadvantage and to provide a continuous measurement method for hydrogen gas concentration enabling reliable measurement of the concentration of hydrogen gas as molecular hydrogen contained in a liquid to be measured.
Another object of the present invention is to provide a hydrogen gas concentration measurement device used in the continuous measurement method for hydrogen gas concentration.

## Solution to Problem

In order to achieve the object, a continuous measurement method for hydrogen gas concentration of the present invention includes a step of placing a hydrogen gas permeable material in such a manner as to come in contact with a flowing hydrogen water containing hydrogen gas and introducing, from the hydrogen water, a hydrogen gas passing through the hydrogen gas permeable material into a hollow part, a step of introducing a first gas substantially inert to hydrogen into the hollow part through a gas supply pump provided on a gas supply conduit connected to the hollow part and extracting and exhausting a hydrogen gas-containing second gas in an amount equal to that of the first gas, from the hollow part through a gas exhaust pump provided on a gas exhaust conduit connected to the hollow part, a step of circulating the hydrogen gas that has passed through the hydrogen gas permeable material and has been introduced into the hollow part from the hydrogen water, through the gas supply conduit into the hollow part by a circulation pump provided on a circulation conduit that is branched from the gas exhaust conduit and is connected to the gas supply conduit, a step of measuring a hydrogen gas concentration in the hollow part when an equilibrium state between a concentration of hydrogen gas contained in the hydrogen water and a concentration of hydrogen gas introduced into the hollow part is achieved, and a step of previously determining a relation between a concentration of hydrogen gas contained in the hydrogen water and a concentration of hydrogen gas introduced into the hollow part and, on the basis of the relation, calculating a concentration of hydrogen gas contained in the hydrogen water from the measured hydrogen gas concentration, wherein said step of measuring the hydrogen gas concentration is carried out in the hollow part at an upstream side from the gas exhaust pump on the gas exhaust conduit.

In the continuous measurement method for hydrogen gas concentration of the present invention, a hydrogen gas permeable material is first placed in such a manner as to come in contact with a hydrogen water containing hydrogen gas. The hydrogen gas contained in the hydrogen water is then allowed to pass through the hydrogen gas permeable material into a hollow part and thus is introduced into the hollow part.

In such a process, a certain equilibrium state is achieved between the concentration of hydrogen gas contained in the hydrogen water and the concentration of hydrogen gas introduced into the hollow part after a while in accordance with a hydrogen gas permeability of the hydrogen gas permeable material.

It may take a long time to achieve the equilibrium state between the concentration of hydrogen gas contained in the hydrogen water and the concentration of hydrogen gas introduced into the hollow part. When changes in concentration of hydrogen gas contained in the hydrogen water are intended to be tracked over time, a change of the hydrogen gas concentration from a low concentration to a high concentration is comparatively easily tracked, whereas a change from a high concentration to a low concentration may be difficult to track.

On this account, in the continuous measurement method for hydrogen gas concentration of the present invention, a first gas substantially inert to hydrogen is next introduced into the hollow part through a gas supply pump provided on a gas supply conduit connected to the hollow part, while a hydrogen gas-containing second gas is extracted and exhausted in an amount equal to that of the first gas, from the hollow part through a gas exhaust pump provided on a gas exhaust conduit connected to the hollow part.

In the continuous measurement method for hydrogen gas concentration of the present invention, the first gas is introduced into the hollow part by the gas supply pump, while the second gas is extracted and exhausted in an amount equal to that of the first gas, from the hollow part by the gas exhaust pump. Hence, the hydrogen gas in the hollow part can be diluted, and the equilibrium state can be achieved at a lower concentration. By diluting the hydrogen gas in the hollow part, changes can be easily tracked even when the concentration of hydrogen gas contained in the hydrogen water changes from a high concentration to a low concentration.

Next, in the measurement method for hydrogen gas concentration of the present invention, the hydrogen gas that has passed through the hydrogen gas permeable material and has been introduced into the hollow part from the hydrogen water is circulated through the gas supply conduit into the hollow part by a circulation pump provided on the circulation conduit. In such a process, the hydrogen gas that has passed through the hydrogen gas permeable material and has been introduced into the hollow part from the hydrogen water can be circulated into the hollow part, and this can stir the gas in the hollow part to equalize the hydrogen gas concentration.

When the equilibrium state between the concentration of hydrogen gas contained in the hydrogen water and the concentration of hydrogen gas introduced into the hollow part is achieved, the hydrogen gas concentration in the hollow part is measured.

Here, the concentration of hydrogen gas contained in the hydrogen water is not necessarily equal to the concentration of hydrogen gas introduced into the hollow part, but a certain correspondence relation is observed in accordance with a hydrogen gas permeability of the hydrogen gas permeable material. By previously determining the correspondence relation, the concentration of hydrogen gas contained in the hydrogen water can be calculated from the measured hydrogen gas concentration on the basis of the correspondence relation.

A hydrogen gas concentration measurement device of the present invention is a hydrogen gas concentration measurement device configured to continuously measure a concentration of hydrogen gas contained in a flowing hydrogen water, and includes a hydrogen gas permeable material placed in such a manner as to come in contact with a hydrogen water containing hydrogen gas, a member forming a hollow part configured to store a hydrogen gas passing through the hydrogen gas permeable material from the hydrogen water, a gas introduction unit configured to introduce a first gas substantially inert to hydrogen into the hollow part, a gas exhaust unit configured to extract and exhaust a hydrogen gas-containing second gas in an amount equal to that of the first gas, from the hollow part, a hydrogen gas concentration detection unit provided in the gas exhaust unit and configured to detect a hydrogen gas concentration in the hollow part, and a gas circulation unit connecting the gas exhaust unit at a downstream side of the hydrogen gas concentration detection unit to the gas introduction unit and configured to circulate a hydrogen gas that has passed through the hydrogen gas permeable material, has been introduced into the hollow part from the hydrogen water, and has extracted from the hollow part, to the gas introduction unit. The gas introduction unit includes a gas supply conduit connected to the hollow part and a gas supply pump provided at a midway point of the gas supply conduit, the gas exhaust unit includes a gas exhaust conduit connected to the hollow part and a gas exhaust pump provided at a midway point of the gas exhaust conduit, the hydrogen gas concentration detection unit is provided at an upstream side from the gas exhaust pump on the gas exhaust conduit, and the circulation unit includes a circulation conduit that is branched from the gas exhaust conduit between the gas exhaust pump and the hydrogen gas concentration detection unit and is connected to the gas supply conduit at a downstream side of the gas supply pump and includes a circulation pump provided at a midway point of the circulation conduit.

With the hydrogen gas concentration measurement device of the present invention, hydrogen gas is allowed to pass through the hydrogen gas permeable material from the hydrogen water into a hollow part formed by the member. When the equilibrium between the concentration of hydrogen gas contained in the hydrogen water and the concentration of hydrogen gas introduced into the hollow part is achieved, the hydrogen gas concentration in the hollow part can be detected by the hydrogen gas concentration detection unit.

With the hydrogen gas concentration measurement device of the present invention, the first gas is introduced into the hollow part by the gas introduction unit, while the second gas is extracted and exhausted in an amount equal to that of the first gas, from the hollow part by the gas exhaust unit. Hence, the hydrogen gas in the hollow part can be diluted, and the equilibrium state can be achieved at a lower concentration. By diluting the hydrogen gas in the hollow part, changes can be easily tracked even when the concentration of hydrogen gas contained in the hydrogen water changes from a high concentration to a low concentration.

The hydrogen gas concentration measurement device of the present invention includes the gas circulation unit, and thus the hydrogen gas that has passed through the hydrogen gas permeable material and has been introduced into the hollow part from the hydrogen water can be circulated through the gas supply conduit into the hollow part. This can stir the gas in the hollow part to equalize the hydrogen gas concentration.

In the device, the hydrogen gas concentration detection unit is provided in the gas exhaust unit, and this structure reduces the effect of the first gas introduced by the gas introduction unit and enables reliable measurement of the hydrogen gas concentration in the hollow part.

A first aspect of the hydrogen gas concentration measurement device of the present invention may include a pipe made from a hydrogen gas permeable material and having an inside configured to allow a hydrogen water to flow and a jacket member covering an outer peripheral surface of the pipe over a predetermined length and forming the hollow part.

With the hydrogen gas concentration measurement device of the first aspect, the hydrogen gas contained in a hydrogen water flowing through the pipe can be allowed to pass through the pipe into the hollow part.

A second aspect of the hydrogen gas concentration measurement device of the present invention may include a side wall member made from a hydrogen gas permeable material and forming a part of a side wall of a storage chamber in which a hydrogen water is to be stored and a jacket member covering an outer surface of the side wall member and forming the hollow part.

In the hydrogen gas concentration measurement device of the second aspect, a part of the side wall of a storage chamber in which a hydrogen water is to be stored is made from a hydrogen gas permeable material, and thus the hydrogen gas contained in a hydrogen water stored in the storage chamber can be allowed to pass through the hydrogen gas permeable material into the hollow part.

A third aspect of the hydrogen gas concentration measurement device of the present invention may include a member made from a hydrogen gas permeable material and forming the hollow part configured to be immersed in a hydrogen water.

With the hydrogen gas concentration measurement device of the third aspect, while the member forming the hollow part is immersed in a hydrogen water, the hydrogen gas concentration in the hydrogen water is measured. In the device, the member forming the hollow part is made from a hydrogen gas permeable material, and thus the hydrogen gas contained in the hydrogen water can be allowed to pass through the hydrogen gas permeable material into the hollow part. By providing the hydrogen gas concentration detection unit in the gas exhaust unit, the hydrogen gas concentration in the hollow part can be measured outside the hydrogen water.

Brief Description of Drawings

FIG. 1 is a schematic cross-sectional view showing a first embodiment of a hydrogen gas concentration measurement device of the present invention.
FIG. 2A is a graph showing time courses of hydrogen gas concentration determined by the hydrogen gas concentration measurement device of the present invention and a dissolved hydrogen concentration measurement device as a standard, and FIG. 2B is a graph after correction of the time lag in FIG. 2A.
FIG. 3 is a graph showing the relation between the measured value by the hydrogen gas concentration measurement device of the present invention and the measured value by the dissolved hydrogen concentration measurement device as a standard shown in FIG. 2B.
FIG. 4 is a schematic cross-sectional view showing a second embodiment of the hydrogen gas concentration measurement device of the present invention.
FIG. 5 is a schematic cross-sectional view showing a third embodiment of the hydrogen gas concentration measurement device of the present invention.

Description of Embodiments

Embodiments of the present invention will next be described in further detail with reference to the accompanying drawings.

A continuous measurement method for hydrogen gas concentration of the embodiment includes a step of placing a hydrogen gas permeable material in such a manner as to come in contact with a flowing hydrogen water containing hydrogen gas and introducing, from the hydrogen water, a hydrogen gas passing through the hydrogen gas permeable material into a hollow part, a step of introducing a first gas substantially inert to hydrogen into the hollow part through a gas supply pump provided on a gas supply conduit connected to the hollow part and extracting and exhausting a hydrogen gas-containing second gas in an amount equal to that of the first gas, from the hollow part through a gas exhaust pump provided on a gas exhaust conduit connected to the hollow part, a step of circulating the hydrogen gas that has passed through the hydrogen gas permeable material and has been introduced into the hollow part from the hydrogen water, through the gas supply conduit into the hollow part by a circulation pump provided on a circulation conduit that is branched from the gas exhaust conduit and is connected to the gas supply conduit, a step of measuring a hydrogen gas concentration in the hollow part when an equilibrium state between a concentration of hydrogen gas contained in the hydrogen water and a concentration of hydrogen gas introduced into the hollow part is achieved, and a step of previously determining a correspondence relation between a concentration of hydrogen gas contained in the hydrogen water and a concentration of hydrogen gas introduced into the hollow part and, on the basis of the correspondence relation, calculating a concentration of hydrogen gas contained in the hydrogen water from the measured hydrogen gas concentration.

The hydrogen gas permeable material is exemplified by silicone rubber, butyl rubber, and polystyrene. The way to place the hydrogen gas permeable material in such a manner as to come in contact with a hydrogen water is exemplified by a method of forming a pipe through which a hydrogen water flows, from the hydrogen gas permeable material, a method of forming a part of the side wall of a storage chamber in which a hydrogen water is stored, from the hydrogen gas permeable material, and a method of forming a hollow body including the hollow part from the hydrogen gas permeable material and immersing the hollow body in a hydrogen water.

In the measurement method for hydrogen gas concentration of the embodiment, the hydrogen gas permeable material is first placed in such a manner as to come in contact with a hydrogen water, and hydrogen gas contained in the hydrogen water is allowed to pass through the hydrogen gas permeable material and is introduced into the hollow part.

In such a process, the hydrogen gas concentration in the hollow part is increased due to the difference between a hydrogen gas permeation amount of the hydrogen gas permeable material and a hydrogen gas permeation amount of a material forming the hollow part. After a while, a certain equilibrium state is achieved between the concentration of hydrogen gas contained in the hydrogen water and the concentration of hydrogen gas introduced into the hollow part in accordance with the hydrogen gas permeability of the hydrogen gas permeable material.

When the equilibrium state is achieved, the hydrogen gas concentration in the hollow part is measured. The hydrogen gas concentration can be measured by a known hydrogen sensor such as a catalytic combustion type hydrogen sensor.

When the equilibrium state is achieved, the concentration of hydrogen gas contained in the hydrogen water is not necessarily equal to the concentration of hydrogen gas introduced into the hollow part, but a certain correspondence relation is observed in accordance with the hydrogen gas permeability of the hydrogen gas permeable material. By previously determining the correspondence relation, the concentration of hydrogen gas contained in a hydrogen water can be calculated from the measured hydrogen gas concentration on the basis of the correspondence relation.

It may take a long time to achieve the equilibrium state between the concentration of hydrogen gas contained in a hydrogen water and the concentration of hydrogen gas introduced into the hollow part.

The hydrogen gas once stored in the hollow part can pass through a member forming the hollow part, but is unlikely to be immediately exhausted to the outside. On this account, when changes in concentration of hydrogen gas contained in a hydrogen water are intended to be tracked over time, a change of the hydrogen gas concentration from a low concentration to a high concentration is comparatively easily tracked, whereas a change from a high concentration to a low concentration may be difficult to track.

In the measurement method for hydrogen gas concentration of the embodiment, a first gas substantially inert to hydrogen is introduced into the hollow part, while a hydrogen gas-containing second gas is extracted and exhausted in an amount equal to that of the first gas, from the hollow part. In addition, the hydrogen gas that has passed through the hydrogen gas permeable material and has been introduced into the hollow part from the hydrogen water is circulated into the hollow part, and when an equilibrium state between the concentration of hydrogen gas contained in the hydrogen water and the concentration of hydrogen gas introduced into the hollow part is achieved, the concentration of the hydrogen gas that has passed through the hydrogen gas permeable material and has been introduced into the hollow part from the hydrogen water can be measured.

The first gas may be any gas that does not substantially react with hydrogen, and air can be used, for example. In this case, the second gas is a mixture gas of air and hydrogen gas.

As mentioned above, by introducing the first gas into the hollow part and extracting and exhausting the second gas in an amount equal to that of the first gas, from the hollow part, the hydrogen gas in the hollow part can be diluted, and the equilibrium state can be achieved at a lower concentration. By diluting the hydrogen gas in the hollow part, changes can be easily tracked even when the concentration of hydrogen gas contained in the hydrogen water changes from a high concentration to a low concentration.

The measurement method for hydrogen gas concentration of the embodiment can be performed with the following hydrogen gas measurement device, for example.

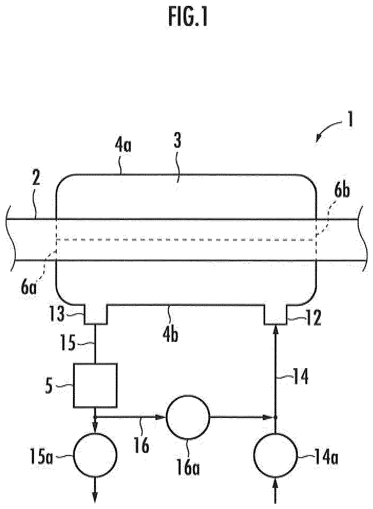

With reference to FIG. 1, a first example of an embodiment of a hydrogen gas measurement device 1 will be described first. The hydrogen gas concentration measurement device 1 includes a pipe 2 made from the hydrogen gas permeable material and configured to allow a hydrogen water to flow, jacket members 4a, 4b covering the entire circumference of the outer peripheral surface of the pipe 2 over a predetermined length and forming a hollow part 3, and a hydrogen gas sensor 5.

The hydrogen gas concentration measurement device 1 includes a gas supply port 12 and a gas exhaust port 13 that are provided on the jacket member 4b. To the gas supply port 12, a gas supply conduit 14 is connected, and at a midway point of the gas supply conduit 14, a gas supply pump 14a is provided. To the gas exhaust port 13, a gas exhaust conduit 15 is connected. At a midway point of the gas exhaust conduit 15, a gas exhaust pump 15a is provided, and at the upstream side of the gas exhaust pump 15a, the hydrogen gas sensor 5 is provided.

The gas exhaust conduit 15 branches out into a circulation conduit 16 between the gas exhaust pump 15a and the hydrogen gas sensor 5, and the circulation conduit 16 is connected to the gas supply conduit 14 at the downstream side of the gas supply pump 14a. At a midway point of the circulation conduit 16, a circulation pump 16a is provided.

In the hydrogen gas concentration measurement device 1, the pipe 2 is formed from the hydrogen gas permeable material, and thus the hydrogen gas permeable material is placed in such a manner as to come in contact with a hydrogen water. The pipe 2 is exemplified by pipes used in various plants, nuclear power plants, hemodialyzers, and the like.

The jacket members 4a, 4b form the hollow part 3 by sandwiching the pipe 2 from the top and bottom therebetween. When forming the hollow part 3, the jacket members 4a, 4b form, at the respective ends, insert ports 6a, 6b through which the pipe 2 is inserted. The jacket members 4a, 4b may be integrated when the pipe 2 can be inserted into the hollow part 3.

As the hydrogen gas sensor 5, a known hydrogen sensor such as a catalytic combustion type hydrogen sensor can be used.

When the hydrogen gas concentration measurement device 1 shown in FIG 1 is used, the pipe 2 is first sandwiched between the jacket members 4a, 4b to cover the entire circumference of the outer peripheral surface of the pipe 2 over a predetermined length, and the hollow part 3 is formed. Here, the pipe 2 is made from a hydrogen gas permeable material, and thus the hydrogen gas contained in a hydrogen water flowing through the pipe 2 passes through the pipe 2 and flows into the hollow part 3.

In the process, in the hydrogen gas concentration measurement device 1, the gas supply pump 14a is activated to supply a first gas substantially inert to hydrogen, such as air, from the gas supply conduit 14 into the hollow part 3, while the gas exhaust pump 15a is activated to exhaust a second gas in an amount equal to that of the air supplied by the gas supply pump 14a, from the hollow part 3. The second gas exhausted by the gas exhaust pump 15a is a mixture gas of hydrogen gas and air.

In such a process, the amount of the hydrogen gas exhausted from the hollow part 3 increases due to the air supplied by the gas supply pump 14a, and the time for achieving the equilibrium between the concentration of hydrogen gas introduced into the hollow part 3 and the concentration of hydrogen gas contained in a hydrogen water flowing through the pipe 2 can be reduced. In addition, the amount of hydrogen gas exhausted from the hollow part 3 increases, and thus even when the concentration of hydrogen gas contained in a hydrogen water flowing through the pipe 2 changes from a high concentration to a low concentration, the change can be easily tracked.

When the circulation pump 16a is activated while the gas supply pump 14a and the gas exhaust pump 15a are stopped, the hydrogen gas that has passed through the pipe 2 and has been introduced into the hollow part 3 from the hydrogen water is circulated through the gas exhaust conduit 15, the circulation conduit 16, and the gas supply conduit 14. This process stirs the hydrogen gas introduced into the hollow part 3 to equalize the concentration thereof. Hence, the hydrogen gas concentration in the hollow part 3 can be more exactly detected.

After a while, the equilibrium between the concentration of hydrogen gas flowing into the hollow part 3 and the concentration of hydrogen gas contained in a hydrogen water flowing through the pipe 2 is achieved. The time for achieving the equilibrium can be determined by detecting a constant hydrogen gas concentration for a predetermined period of time when the hydrogen gas concentration in the hollow part 3 is detected over time by the hydrogen gas sensor 5.

When the equilibrium between the hydrogen gas concentration in the hollow part 3 and the concentration of hydrogen gas contained in the hydrogen water is achieved, the hydrogen gas concentration in the hollow part 3 is detected by the hydrogen gas sensor 5. When the equilibrium is achieved, the hydrogen gas concentration in the hollow part 3 is a certain concentration determined by the hydrogen gas permeability of the pipe 2, and has a certain correspondence relation to the concentration of hydrogen gas contained in a hydrogen water flowing through the pipe 2.

Hence, by previously determining the correspondence relation between the concentration of hydrogen gas contained in a hydrogen water flowing through the pipe 2 and the hydrogen gas concentration in the hollow part 3, the concentration of hydrogen gas contained in a hydrogen water can be indirectly determined from the hydrogen gas concentration in the hollow part 3 detected by the hydrogen gas sensor 5.

The hydrogen gas concentration measurement device 1 can measure the concentration of hydrogen gas contained in a hydrogen water flowing through a pipe 2 used in various plants, nuclear power plants, hemodialyzers, and the like, as mentioned above.

In various plants, a hydrogen water flowing through a pipe 2 can be sampled to measure the hydrogen gas concentration, but this case has a problem of failing to continuously measure the concentration. In addition, the sampled hydrogen water is discarded and becomes unavailable. It may be difficult to sample the hydrogen water itself when the concentration of hydrogen gas contained in a hydrogen water flowing through a pipe 2 is measured in a nuclear power plant, for example.

By providing a hydrogen sensor or inserting the probe of a hydrogen sensor in a pipe 2, the hydrogen gas concentration can be measured without sampling of the hydrogen water. However, the case of a dialysate for hemodialysis and the like has a problem of contamination of a dialysate due to the contact with the hydrogen sensor or the probe thereof.

The hydrogen gas concentration measurement device 1 of the embodiment advantageously enables noncontact measurement of the concentration of hydrogen gas contained in a hydrogen water flowing through the pipe 2 without sampling of the hydrogen water or without contact with the hydrogen water.

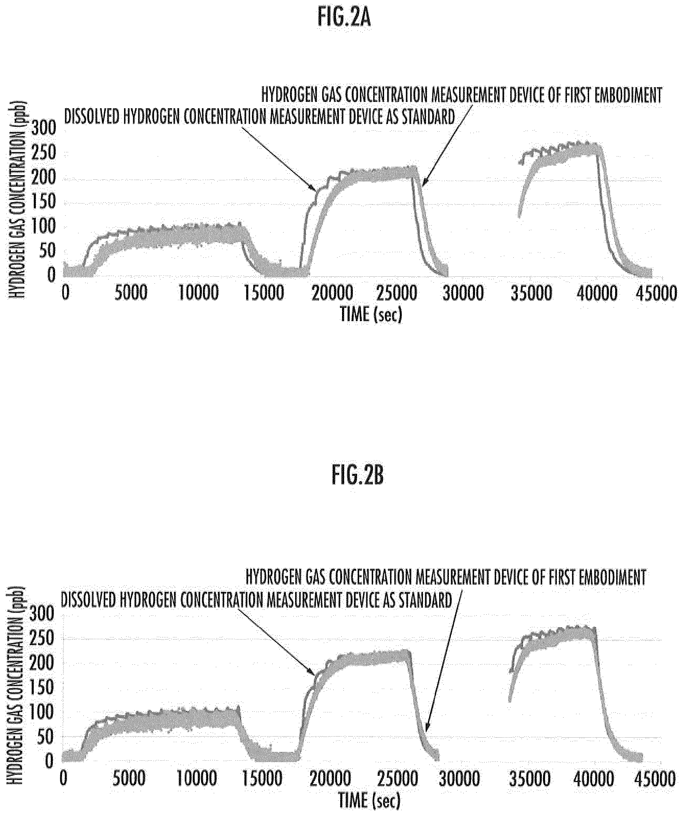

Next, a hydrogen water was allowed to flow through a silicone rubber pipe 2 having an outer diameter of 12 mm and an inner diameter of 6 mm, and the concentration of hydrogen gas contained in the hydrogen water was measured with the hydrogen gas concentration measurement device 1 and with a dissolved hydrogen concentration measurement device (manufactured by DKK-TOA Corporation, trade name: DH-35K) as a standard. The time courses of the hydrogen gas concentration determined by the devices are shown in FIG. 2A. The dissolved hydrogen concentration measurement device as the standard directly measures the concentration of hydrogen gas contained in the hydrogen water by diaphragm polarography.

FIG. 2A indicates that the measured value by the hydrogen gas concentration measurement device 1 has a time lag of about 10 minutes with respect to the measured value by the dissolved hydrogen concentration measurement device as the standard, but shows an identical change tendency, and satisfactory tracks changes in the concentration of hydrogen gas contained in the hydrogen water flowing through the pipe 2.

Next, the result after correction of the time lag in FIG. 2A is shown in FIG. 2B.

FIG. 2B indicates that when the time lag is considered, the measured value by the hydrogen gas concentration measurement device 1 is in good agreement with the measured value by the dissolved hydrogen concentration measurement device as the standard.

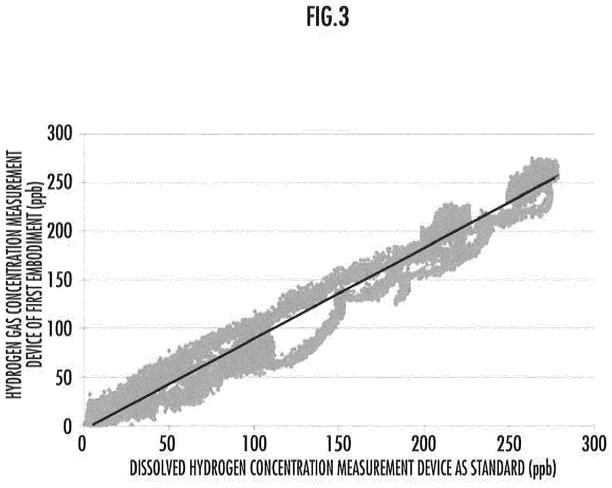

Next, the relation between the measured value by the hydrogen gas concentration measurement device 1 and the measured value by the dissolved hydrogen concentration measurement device as the standard with consideration of time lag is shown in FIG. 3. FIG. 3 indicates that the following relation is satisfied therebetween where y is the measured value by the hydrogen gas concentration measurement device 1, and x is the measured value by the dissolved hydrogen concentration measurement device as the standard.

$$y = 0.9355x - 3.7854$$

Coefficient of correlation, $R^2 = 0.9788$

The hydrogen gas concentration measurement device 1 of the embodiment enables determination of the concentration of hydrogen gas contained in a hydrogen water flowing through the pipe 2 from the hydrogen gas concentration in the hollow part 3 detected by the hydrogen gas sensor 5 on the basis of the correspondence relation shown in FIG. 3. It is thus obvious that the hydrogen gas concentration measurement device 1 of the embodiment enables noncontact measurement without sampling of a hydrogen water flowing through the pipe 2 or without contact with the hydrogen water.

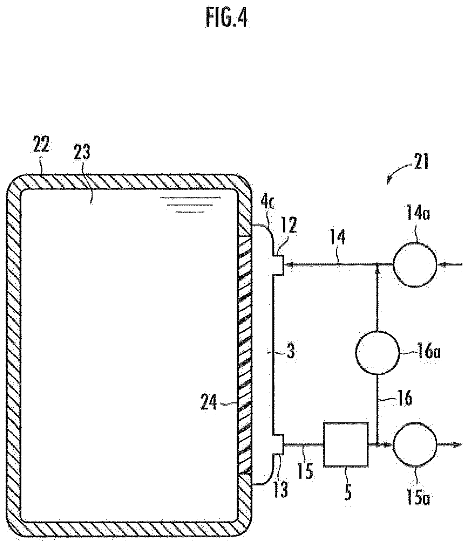

A second example of the embodiment of the hydrogen gas measurement device 21 will next be described with reference to FIG. 4. In the hydrogen gas concentration measurement device 21, a part of the side wall of a storage chamber 22 becomes a side wall member 24 made from the hydrogen gas permeable material with respect the hydrogen water 23 stored in the storage chamber 22, and thus the hydrogen gas permeable material is placed in such a manner as to come in contact with the hydrogen water 23.

The hydrogen gas concentration measurement device 21 includes a jacket member 4c that covers the side wall member 24 to form a hollow part 3 and includes a gas supply port 12 and a gas exhaust port 13 that are provided on the jacket member 4c. To the gas supply port 12, a gas supply conduit 14 is connected, and at a midway point of the gas supply conduit 14, a gas supply pump 14a is provided. To the gas exhaust port 13, a gas exhaust conduit 15 is connected. At a midway point of the gas exhaust conduit 15, a gas exhaust pump 15a is provided, and at the upstream side of the gas exhaust pump 15a, a hydrogen gas sensor 5 is provided.

The gas exhaust conduit 15 branches out into a circulation conduit 16 between the gas exhaust pump 15a and the hydrogen gas sensor 5, and the circulation conduit 16 is connected to the gas supply conduit 14 at the downstream side of the gas supply pump 14a. At a midway point of the circulation conduit 16, a circulation pump 16a is provided.

The hydrogen gas concentration measurement device 21 shown in FIG. 4 is completely the same as the hydrogen gas concentration measurement device 1 shown in FIG. 1 except that the hydrogen gas contained in the hydrogen water 23 stored in the storage chamber 22 passes through the side wall member 24 and is introduced into the hollow part 3, and can measure the concentration of the hydrogen gas. The storage chamber 22 is exemplified by a tank for storing hot water used for a footbath or the like.

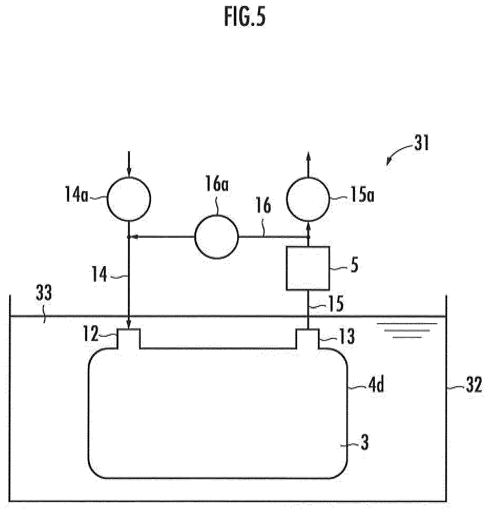

A third example of the embodiment of the hydrogen gas measurement device 31 will next be described with reference to FIG. 5. In the hydrogen gas concentration measurement device 31, a hollow body 4d made from the hydrogen gas permeable measurement device 31, a hollow body 4d made from the hydrogen gas permeable material is immersed in a hydrogen water 33 stored in a storage chamber 32, and thus the hydrogen gas permeable material is placed in such a manner as to come in contact with the hydrogen water 33.

The hydrogen gas concentration measurement device 31 includes a hollow body 4d that forms a hollow part 3 therein and includes a gas supply port 12 and a gas exhaust port 13 that are provided on the hollow body 4d. To the gas supply port 12, a gas supply conduit 14 is connected, and at a midway point of the gas supply conduit 14, a gas supply pump 14a is provided. To the gas exhaust port 13, a gas exhaust conduit 15 is connected. At a midway point of the gas exhaust conduit 15, a gas exhaust pump 15a is provided, and at the upstream side of the gas exhaust pump 15a, a hydrogen gas sensor 5 is provided.

The gas exhaust conduit 15 branches out into a circulation conduit 16 between the gas exhaust pump 15a and the hydrogen gas sensor 5, and the circulation conduit 16 is connected to the gas supply conduit 14 at the downstream side of the gas supply pump 14a. At a midway point of the circulation conduit 16, a circulation pump 16a is provided.

The hydrogen gas concentration measurement device 31 shown in FIG. 5 is completely the same as the hydrogen gas concentration measurement device 1 shown in FIG. 1 except that the hydrogen gas contained in the hydrogen water 33 stored in the storage chamber 32 passes through the hollow body 4d and is introduced into the hollow part 3, and can measure the concentration of the hydrogen gas. The storage chamber 32 is exemplified by a bathtub used for a footbath or the like.

Description of Reference Numerals

| 1, 21, 31 | hydrogen gas concentration measurement device |
| 2 | pipe |
| 3 | hollow part |
| 4a, 4b, 4c | jacket member |
| 4d | hollow body |
| 5 | hydrogen gas sensor |
| 12 | gas supply port |
| 13 | gas exhaust port |
| 14 | gas supply conduit |
| 14a | gas supply pump |
| 15 | gas exhaust conduit |
| 15a | gas exhaust pump |
| 16 | circulation conduit |
| 16a | circulation pump |

**Claims**

1. A continuous measurement method for hydrogen gas concentration, the method comprising:

   a step of placing a hydrogen gas permeable material in such a manner as to come in contact with a flowing hydrogen water containing hydrogen gas and introducing, from the hydrogen water, a hydrogen gas passing through the hydrogen gas permeable material into a hollow part (3);
   a step of introducing a first gas substantially inert to hydrogen into the hollow part (3) through a gas supply pump (14a) provided on a gas supply conduit (14) connected to the hollow part (3) and extracting and exhausting a hydrogen gas-containing gas as a second gas in an amount equal to that of the first gas, from the hollow part (3) through a gas exhaust pump (15a) provided on a gas exhaust conduit (15) connected to the hollow part (3);
   a step of circulating the hydrogen gas that has passed through the hydrogen gas permeable material and has been introduced into the hollow part (3) from the hydrogen water, through the gas supply conduit (14) into the hollow part (3) by a circulation pump (16a) provided on a circulation conduit (16) that is branched from the gas exhaust conduit (15) and is connected to the gas supply conduit (14);

a step of measuring a hydrogen gas concentration in the hollow part (3) when an equilibrium state between a concentration of hydrogen gas contained in the hydrogen water and a concentration of hydrogen gas introduced into the hollow part (3) is achieved; and

a step of previously determining a relation between a concentration of hydrogen gas contained in the hydrogen water and a concentration of hydrogen gas introduced into the hollow part and, based on said relation, calculating a concentration of hydrogen gas contained in the hydrogen water from the measured hydrogen gas concentration, wherein said step of measuring the hydrogen gas concentration is carried out at an upstream side from the gas exhaust pump (15a) on the gas exhaust conduit (15).

2. A hydrogen gas concentration measurement device (1, 21, 31) configured to continuously measure a concentration of hydrogen gas contained in a flowing hydrogen water, the device comprising:

a hydrogen gas permeable material placed in such a manner as to come in contact with a hydrogen water containing hydrogen gas;

a member forming a hollow part (3) configured to store a hydrogen gas passing through the hydrogen gas permeable material from the hydrogen water;

a gas introduction unit configured to introduce a first gas substantially inert to hydrogen into the hollow part (3);

a gas exhaust unit configured to extract and exhaust a hydrogen gas-containing gas as a second gas in an amount equal to that of the first gas, from the hollow part (3);

a hydrogen gas concentration detection unit provided in the gas exhaust unit and configured to detect a hydrogen gas concentration in the hollow part (3); and

a gas circulation unit connecting the gas exhaust unit at a downstream side of the hydrogen gas concentration detection unit to the gas introduction unit and configured to circulate a hydrogen gas that has passed through the hydrogen gas permeable material, has been introduced into the hollow part (3) from the hydrogen water, and has been extracted from the hollow part (3), to the gas introduction unit,

the gas introduction unit including a gas supply conduit (14) connected to the hollow part (3) and a gas supply pump (14a) provided at a midway point of the gas supply conduit (14),

the gas exhaust unit including a gas exhaust conduit (15) connected to the hollow part (3) and a gas exhaust pump (15a) provided at a midway point of the gas exhaust conduit (15),

the circulation unit including a circulation conduit (16) that is branched from the gas exhaust conduit (15) between the gas exhaust pump (15a) and the hydrogen gas concentration detection unit and is connected to the gas supply conduit (14) at a downstream side of the gas supply pump (14a) and including a circulation pump (16a) provided at a midway point of the circulation conduit (16), the hydrogen gas concentration detection unit being provided at an upstream side from the gas exhaust pump (15a) on the gas exhaust conduit (15).

3. The hydrogen gas concentration measurement device (1, 21, 31) according to claim 2, comprising a pipe (2) made from the hydrogen gas permeable material and having an inside configured to allow a hydrogen water to flow, and a jacket member (4a, 4b, 4c) covering an outer peripheral surface of the pipe (2) over a predetermined length and forming the hollow part (3).

4. The hydrogen gas concentration measurement device (1, 21, 31) according to claim 2, comprising a side wall member made from the hydrogen gas permeable material and forming a part of a side wall of a storage chamber in which a hydrogen water is to be stored, and a jacket member (4a, 4b, 4c) covering an outer surface of the side wall member and forming the hollow part (3).

5. The hydrogen gas concentration measurement device (1, 21, 31) according to claim 2, comprising a member made from the hydrogen gas permeable material and forming the hollow part (3) configured to be immersed in a hydrogen water.

**Patentansprüche**

1. Kontinuierliches Verfahren zum Messen einer Wasserstoffgaskonzentration, wobei das Verfahren Folgendes umfasst:

eine Stufe eines Platzierens eines für Wasserstoffgas permeablen Materials in einer solchen Weise, dass es mit einem strömenden, Wasserstoff umfassenden Wasser, das Wasserstoffgas enthält, in Kontakt gelangt, und eines Einführens eines aus dem Wasserstoff umfassenden Wasser (herrührenden) Wasserstoffgases, das durch das für Wasserstoff permeable Material hindurchtritt, in einen Hohlkörper (3);

eine Stufe eines Einführens eines ersten Gases, welches im Wesentlichen gegenüber Wasserstoff inert ist, in den Hohlkörper (3) mittels einer Gaszuführpumpe (14a), die an einer mit dem Hohlkörper (3) verbundenen Gaszuführleitung (14) vorgesehen ist, und eines Extrahierens und Ausströmenlassens eines Wasserstoffgas enthaltenden Gases in Form eines zweiten Gases in einer Menge, die der des ersten Gases entspricht, aus dem Hohlkörper (3) mittels einer Gasauslasspumpe (15a), die an einer Gasauslassleitung (15), welche mit dem Hohlkörper (3) verbunden ist, vorgesehen ist;

eine Stufe eines Zirkulierenlassens des Wasserstoffgases, das durch das für Wasserstoffgas permeable Material hindurchgetreten ist und aus dem Wasserstoff umfassenden Wasser in den Hohlkörper (3) eingeführt worden ist, durch die Gaszuführleitung (14) in den Hohlkörper (3) mittels einer Zirkulationspumpe (16a), die an einer Zirkulationsleitung (16), die von der Gasauslassleitung (15) abzweigt und mit der Gaszuführleitung (14) verbunden ist, vorgesehen ist;

eine Stufe eines Messens einer Wasserstoffgaskonzentration in dem Hohlkörper (3) wenn ein Gleichgewichtszustand zwischen einer Konzentration des in dem Wasserstoff umfassenden Wasser enthaltenen Wasserstoffgases und einer Konzentration des in den Hohlkörper (3) eingeführten Wasserstoffgases erreicht ist; und eine Stufe einer vorher erfolgenden Bestimmung einer Beziehung zwischen einer Konzentration des in dem Wasserstoff umfassenden Wasser enthaltenen Wasserstoffgases und einer Konzentration des in den Hohlkörper eingeführten Wasserstoffgases und, basierend auf dieser Beziehung, eines Berechnens einer Konzentration des in dem Wasserstoff umfassenden Wasser enthaltenen Wasserstoffgases aus der gemessenen Wasserstoffgaskonzentration, wobei die Stufe des Messens der Wasserstoffgaskonzentration an einer bezüglich der Gasauslasspumpe (15a) stromaufseitigen Seite an der Gasauslassleitung (15) durchgeführt wird.

2. Vorrichtung zum Messen einer Wasserstoffgaskonzentration (1, 21, 31), die für eine kontinuierliche Messung einer Konzentration von Wasserstoffgas, das in einem strömenden Wasserstoff umfassenden Wasser enthalten ist, ausgestaltet ist, wobei die Vorrichtung Folgendes umfasst:

ein für Wasserstoffgas permeables Material, das in einer solchen Weise platziert ist, dass es mit einem Wasserstoffgas enthaltenden, Wasserstoff umfassenden Wasser in Kontakt gelangt; ein einen Hohlkörper (3) bildendes Element, das zum Speichern eines aus dem Wasserstoff umfassenden Wasser durch das für Wasserstoffgas permeable Material hindurchtretenden Wasserstoffgases ausgestaltet ist;

eine Gaseinführungseinheit, die zum Einführen eines ersten, gegenüber Wasserstoff im Wesentlichen inerten Gases in den Hohlkörper (3) ausgestaltet ist;

eine Gasauslasseinheit, die für ein Extrahieren und Ablassen eines Wasserstoffgas enthaltenden Gases in Form eines zweiten Gases aus dem Hohlkörper (3) in einer Menge, die der des ersten Gases entspricht, ausgestaltet ist;

eine Einheit zum Detektieren/Nachweis der Wasserstoffgaskonzentration, die in der Gasauslasseinheit vorgesehen ist und zur Detektion einer Wasserstoffgaskonzentration in dem Hohlkörper (3) ausgestaltet ist; und

eine Gaszirkulationseinheit, die die Gasauslasseinheit an einer stromabseitigen Seite der Detektionseinheit der Wasserstoffgaskonzentration mit der Gaseinführeinheit verbindet und zum Zirkulieren eines Wasserstoffgases, das durch das für Wasserstoffgas permeable Material hindurchgetreten ist, aus dem Wasserstoff umfassenden Wasser in den Hohlkörper (3) eingeführt worden ist und aus dem Hohlkörper (3) extrahiert wurde, zu der Gaseinführungseinheit ausgestaltet ist, wobei

die Gaseinführungseinheit eine Gaszuführleitung (14), die mit dem Hohlkörper (3) verbunden ist, und eine Gaszuführpumpe (14a), die an einem mittleren Punkt der Gaszuführleitung (14) vorgesehen ist, umfasst,

die Gasauslasseinheit eine Gasauslassleitung (15), die mit dem Hohlkörper (3) verbunden ist, und eine Gasauslasspumpe (15a), die an einem mittleren Punkt der Gasauslassleitung (15) vorgesehen ist, umfasst,

die Zirkulationseinheit eine Zirkulationsleitung (16), die von der Gasauslassleitung (15) zwischen der Gasauslasspumpe (15a) und der Detektionseinheit der Wasserstoffgaskonzentration abzweigt und mit der Gaszuführleitung (14) an einer stromabseitigen Seite der Gaszuführpumpe (14a) verbunden ist, umfasst und eine Zirkulationspumpe (16a), die an einem mittleren Punkt der Zirkulationsleitung (16) vorgesehen ist, umfasst,

wobei die Detektionseinheit der Wasserstoffgaskonzentration bezüglich der Gasauslasspumpe (15a) an einer stromaufseitigen Seite an der Gasauslassleitung (15) vorgesehen ist.

3. Vorrichtung zum Messen der Wasserstoffgaskonzentration (1, 21, 31) gemäß Anspruch 2, die ein Rohr (2), das aus dem für Wasserstoffgas permeablen Material hergestellt ist und eine Innenseite aufweist, die so ausgestaltet ist, das sie ein Strömen von Wasserstoff umfassendem Wasser ermöglicht, und ein Gehäuseelement (4a, 4b, 4c), das eine äußere periphere Oberfläche des Rohrs (2) über eine festgelegte Länge bedeckt und den Hohlkörper (3) bildet, umfasst.

4. Vorrichtung zum Messen der Wasserstoffgaskonzentration (1, 21, 31) gemäß Anspruch 2, die ein Seitenwandelement, das aus dem für Wasserstoffgas permeablen Material hergestellt ist und einen Teil einer Seitenwand einer Speicherkammer bildet, in der ein Wasserstoff umfassendes Wasser zu speichern ist, und ein Gehäuseelement (4a, 4b, 4c), das eine äußere Oberfläche des Seitenwandelements bedeckt und den Hohlkörper (3) bildet, umfasst.

5. Vorrichtung zum Messen der Wasserstoffgaskonzentration (1, 21, 31) gemäß Anspruch 2, die ein Element umfasst, das aus dem für Wasserstoffgas permeablen Material hergestellt ist und den Hohlkörper (3) bildet, der so ausgestaltet ist, dass er in ein Wasserstoff umfassenden Wasser eingetaucht werden kann.

**Revendications**

1. Procédé de mesure continue pour concentration en gaz hydrogène, le procédé comprenant:

   une étape de placement d'un matériau perméable au gaz hydrogène de telle manière qu'il vienne en contact avec une eau hydrogénée s'écoulant contenant du gaz hydrogène et l'introduction, à partir de l'eau hydrogénée, d'un gaz hydrogène passant à travers le matériau perméable au gaz hydrogène dans une partie creuse (3);
   une étape d'introduction d'un premier gaz essentiellement inerte à l'hydrogène dans la partie creuse (3) à travers une pompe d'approvisionnement en gaz (14a) fournie sur une conduite d'approvisionnement en gaz (14) connectée à la partie creuse (3) et extrayant et faisant s'échapper un gaz contenant du gaz hydrogène comme second gaz en une quantité égale à la cellule du premier gaz, de la partie creuse (3) à travers une pompe d'échappement de gaz (15a) fournie sur une conduite d'échappement de gaz (15) connectée à la partie creuse (3);
   une étape de circulation du gaz hydrogène qui est passé à travers le matériau perméable au gaz hydrogène et a été introduit dans la partie creuse (3) de l'eau hydrogénée, à travers la conduite d'approvisionnement de gaz (14) dans la partie creuse (3) par une pompe de circulation (16a) fournie sur une conduite de circulation (16) qui est branchée à partir de la conduite d'échappement de gaz (15) et est connectée à la conduite d'approvisionnement de gaz (14);
une étape de mesure de concentration en gaz hydrogène dans la partie creuse (3) quand un état d'équilibre entre une concentration de gaz hydrogène contenue dans l'eau hydrogénée et une concentration de gaz hydrogène introduit dans la partie creuse (3) est atteinte; et
une étape de détermination précédemment d'une relation entre une concentration de gaz hydrogène contenu dans l'eau hydrogénée et une concentration de gaz hydrogène introduit dans la partie creuse et, sur la base de ladite relation, le calcul d'une concentration de gaz hydrogène contenue dans l'eau hydrogénée à partir de la concentration en gaz hydrogène mesurée,
ladite étape de mesure de la concentration en gaz hydrogène est réalisée à un côté en amont de la pompe d'échappement de gaz (15a) sur la conduite d'échappement de gaz (15).

2. Dispositif de mesure de concentration en gaz hydrogène (1, 21, 31) configuré pour mesurer en continu une concentration de gaz hydrogène contenu dans une eau hydrogénée s'écoulant, le dispositif comprenant:

   un matériau perméable au gaz hydrogène placé de telle manière qu'il vienne en contact avec un eau hydrogénée contenant du gaz hydrogène;
   un élément formant une partie creuse (3) configuré pour stocker un gaz hydrogène passant à travers le matériau perméable au gaz hydrogène de l'eau hydrogénée;
   une unité d'introduction de gaz configurée pour introduire un premier gaz essentiellement inerte à l'hydrogène dans la partie creuse (3);
   une unité d'échappement de gaz configurée pour extraire et faire échapper un gaz contenant du gaz hydrogène comme second gaz en une quantité égale à celle du premier gaz, à partir de la partie creuse (3);
   une unité de détection de concentration en gaz hydrogène disposée dans l'unité d'échappement de gaz et configurée pour détecter une concentration en gaz hydrogène dans la partie creuse (3); et
   une unité de circulation de gaz connectant l'unité d'échappement de gaz à un côté en aval de l'unité de détection de concentration en gaz hydrogène à l'unité d'introduction de gaz et configurée pour faire circuler un gaz hydrogène qui est passé à travers le matériau perméable au gaz hydrogène, a été introduit dans la partie creuse (3) de l'eau hydrogénée, et a été extrait de la partie creuse (3), à l'unité d'introduction de gaz,
   l'unité d'introduction de gaz comprenant une conduite d'approvisionnement de gaz (14) connectée à la partie creuse (3) et une pompe d'approvisionnement de gaz (14a) fournie à un point à mi-chemin de la conduite d'approvisionnement de gaz (14),

l'unité d'échappement de gaz comprenant une conduite d'échappement de gaz (15) connectée à la partie creuse (3) et une pompe d'échappement de gaz (15a) fournie à un point à mi-chemin de la conduite d'échappement de gaz (15),

l'unité de circulation comprenant une conduite de circulation (16) qui est branchée à partir de la conduite d'échappement de gaz (15) entre la pompe d'échappement de gaz (15a) et l'unité de détection de concentration en gaz hydrogène et est connectée à une conduite d'approvisionnement de gaz (14) à un côté en aval de la pompe d'approvisionnement de gaz (14a) et comprenant une pompe de circulation (16a) fournie à un point à mi-chemin de la conduite de circulation (16),

l'unité de détection de concentration en gaz hydrogène étant fournie à un côté en amont de la pompe d'échappement de gaz (15a) sur la conduite d'échappement de gaz (15).

3. Dispositif de mesure de concentration en gaz hydrogène (1, 21, 31) selon la revendication 2, comprenant un tuyau (2) constitué du matériau perméable au gaz hydrogène et ayant un intérieur configuré pour permettre à une eau hydrogénée de s'écouler, et un élément d'enveloppe (4a, 4b, 4c) recouvrant une surface périphérique externe du tuyau (2) sur une longueur prédéterminée et formant la partie creuse (3).

4. Dispositif de mesure de concentration en gaz hydrogène (1, 21, 31) selon la revendication 2, comprenant un élément de paroi latérale constitué du matériau perméable au gaz hydrogène et formant une partie d'une paroi latérale d'une chambre de stockage dans laquelle de l'eau hydrogénée doit être stockée, et un élément d'enveloppe (4a, 4b, 4c) recouvrant une surface externe de l'élément de paroi latérale et formant la partie creuse (3).

5. Dispositif de mesure de concentration en gaz hydrogène (1, 21, 31) selon la revendication 2, comprenant un élément constitué du matériau perméable au gaz hydrogène et formant la partie creuse (3) configurée pour être immergée dans une eau hydrogénée.

FIG.1

# FIG.2A

# FIG.2B

# FIG.3

FIG.4

## FIG.5

**EP 3 312 584 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012163531 A
- WO 2015059810 A1
- WO 2015074990 A1
- JP H085618 A